# EUROPEAN PATENT SPECIFICATION

(11) **EP 2 686 661 B1**
(45) Date of publication and mention of the grant of the patent: **18.10.2017**
(21) Application number: 12714976.3
(22) Date of filing: 14.03.2012
(51) Int. Cl.: G01N 1/30, G01N 33/569

(54) **METHOD, COMPOSITION AND KIT FOR VISUALIZATION AND CHARACTERIZATION OF THE NERVOUS SYSTEM**
VERFAHREN, ZUSAMMENSETZUNG UND KIT ZUR VISUALISIERUNG UND CHARAKTERISIERUNG DES NERVENSYSTEMS
PROCEDE, COMPOSITION ET KIT POUR LA VISUALISATION ET CHARACTERISATION DU SYSTEME NERVEUX

(30) Priority: 16.03.2011 IT PD20110081
(43) Date of publication of application: 22.01.2014
(73) Proprietor: UNIVERSITA' DEGLI STUDI DI CAGLIARI, 09124 Cagliari (IT); UNIVERSITA' DEGLI STUDI DI SASSARI, 07100 Sassari (IT)
(72) Inventor: SPIGA, Saturnino, I-09044 Quartucciu (IT); DIANA, Marco, I-09012 Capoterra (IT)
(74) Representative: Gervasi, Gemma
(86) International application number: PCT/EP2012/054454
(87) International publication number: WO 2012/123492

(56) References cited:
- WO-A1-96/00234
- WO-A2-2007/106402
- JP-A- 2008 237 072
- PROCEEDINGS OF THE NATIONAL ACADEMY OF SCIENCES OF THE UNITED STATES OF AMERICA NATIONAL ACADEMY OF SCIENCES OF THE UNITED STATES OF AMERICA USA, vol. 103, no. 9, 28 February 2006 (2006-02-28), pages 3399-3404, XP002662411, ISSN: 0027-8424, DOI: DOI:10.1073/PNAS.0511244103

## Description

### Field of the invention

The invention is related to a method of visualization and characterization of the nervous system and, more specifically, of the central nervous system, by staining the same histological section for metal impregnation and immunocytochemistry. The doubly stained histological sections are subsequently subjected to a qualitative and quantitative morphological and antigenic and/or neurochemical analysis, by means of conventional optical microscopy or confocal laser scanning microscopy.

### State of the art

The histological method of metal impregnation by applying potassium dichromate and silver nitrate developed by Camillo Golgi in 1873 continues, with all its subsequent variations, to still represent a method of reference today, and one essential for the histological analysis of the nervous tissue and, more specifically, of the central nervous system. At present the most used metal impregnation is the one proposed by Cox in 1891, known as mercuric impregnation or Golgi-Cox's method or black reaction, which foresees replacement of silver nitrate with mercuric chloride. This technique is widely used for the qualitative analysis of neuron morphology and in determining the quantity of dendritic spines, and the length and complexity of the dendrite branching.

The international application WO9600234 A1 discloses a fixative comprising both, a precipitating fixative including Golgi-type of fixatives and cross-linking fixatives such as formaldehyde. Another successful technique applied to the visualization and characterization of tissues is immunohistochemistry, a technique which exploits the ability of antibodies labeled with suitable markers, preferably fluorescent, to bind specific antigens with high affinity. This technique is widely used to localize antigens on both a cellular and sub-cellular level, and it is widely applied on the central nervous system as well.

Both of these two methodologies of visualization and characterization of tissues have in recent years been improved by applying confocal laser scanning microscopy (CLSM) using reflection mode, the latter a technique suitable for the modeling and reconstruction in 3D of cellular structures, especially neurons. The very extensive use made recently of confocal laser scanning microscopy applied to these techniques is due to the possibility of obtaining top-quality images from specimens prepared for examination under conventional or fluorescent light. Confocal reflection microscopy, in fact, allows, in the case of the mercuric impregnation technique, to visualize the tissues impregnated with the metal particles in an advantageous manner, because the metal particles reflect the laser light and do not display a photo-bleaching phenomenon. In the case, instead, of immunohistochemistry, this analytical method for tissues allows to control field depth, to reduce background noise, and to collect serial optical sections from the specimen. Furthermore, confocal laser scanning microscopy allows to survey fine details which can represent substantial information and which would be lost with conventional microscopy.

In the past, some attempts have been made to combine metal impregnation and labeling with the immunoperoxidase enzyme. In this regard, the Golgi impregnation methods after retrograde transport of the peroxidase, initially proposed by Somogyi et al. and Freund and Somogyi, respectively applied to the visual cortex of a cat (Somogyi P et al., 1983, Neuroscience, 9, 475-490) and of a monkey (Freund TF, Somogyi P, 1983, Neuroscience 9, 463-474), can be remembered. However, the methods proposed are considerably complex and poorly reproducible.

Techniques have also been proposed for tissue perfusion and fixation that are such so as to make Golgi's method and its variations more versatile, depending on the experimental aims. With regard to fixation, several times have been proposed for the fixation of tissues in formalin, from a few hours (Williams RS, 1983, J. Neuropath. Exp. Neurol. 42, 210-212) to several years (Goradz R et al., 2003, J. Neuroscience Meth. 131, 1-7).

In no case, instead, the combination of double staining with the Golgi-Cox mercuric impregnation method and immunofluorescence has been proposed. This is presumably due to the fact that the metal impregnation reaction and immunofluorescence are deemed incompatible, due to the significant differences in the perfusion and fixation procedures foreseen for the two techniques (Lee KW et al., 2006, Proc. Natl. Acad. Sci. USA 28, 103, 3399-3404).

### Summary

Therefore, to remedy the drawbacks of the methods in use, a purpose of this invention is to develop a method of visualization and characterization of nervous system tissues and, more specifically, of the central nervous system, by staining the same histological section for metal impregnation and immunocytochemistry. In particular, the purpose of this invention is to develop a method simple to perform and highly reproducible, which allows scientists to simultaneously obtain an amplification of the effects of the two methods for visualization of the morpho-structural conformations of nervous tissues and a synergism between them, allowing them to analyze the same histological sections by means of conventional optical microscopy or confocal laser scanning microscopy.

The method described hereinafter is, therefore, based on combining the two histological staining techniques in the same tissue specimens to be analyzed, that is, metal impregnation and immunohistochemistry, associated to the analysis of the specimens thus prepared with conventional optical microscopy or, preferably, with confocal laser scanning microscopy.

The solution found to overcome the incompatibility among the different metal impregnation procedures, with special regard to Golgi-Cox impregnation but not limited to this metal impregnation, and immunohistochemistry, essentially consists in the fixing procedures of the tissues to be studied, which foresees fixation of tissues *ex vivo* using appropriate concentrations of paraformaldehyde, optionally pre-fixed with the same *ex vivo* fixing solution comprising paraformaldehyde. Therefore, the object of this invention is a method of visualization and characterization of the nervous system by combining staining for metal impregnation and immunocytochemistry, comprising at least the steps of:
- fixing a nervous tissue samples with an aqueous solution comprising paraformaldehyde in a concentration comprised from 1 to 4% w/v;
- subjecting the fixed tissue samples to metal impregnation;
- treating the sections of metal impregnated tissues with suitable reagents for the intended immunohistochemistry staining.

The method according to the invention finds application in the experimental and clinical field for the study of the characteristics of neuronal connections in the central nervous system.

The method and its possible embodiments, as also its advantages, will be made clearer by the detailed description of the invention which follows, with the aid of the figures annexed to it.

### Brief description of the figures

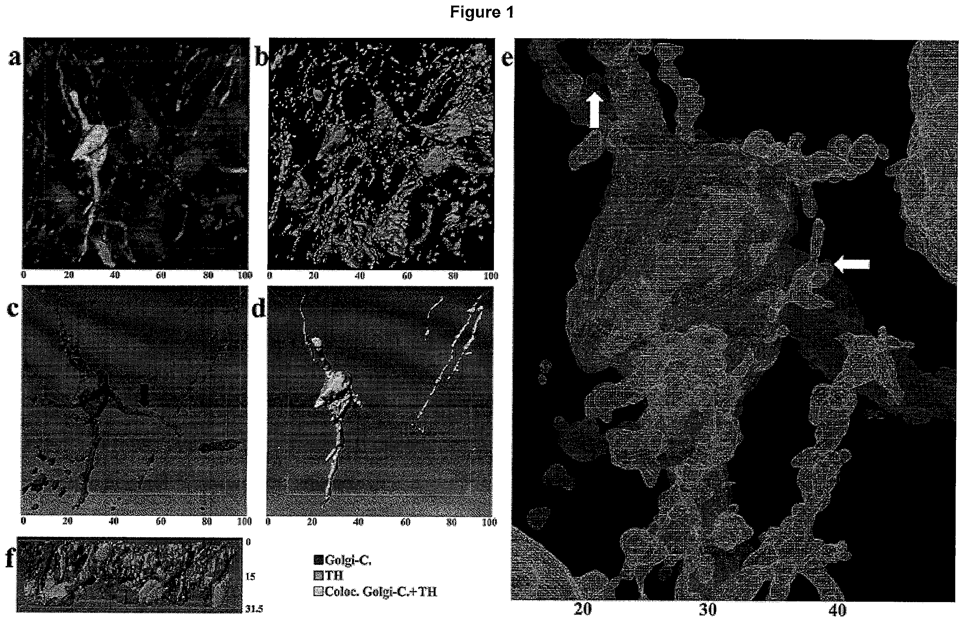

Figure 1. The figure shows the volume rendering of some neurons of the Substantia Nigra compacta (SNc) impregnated with the Golgi-Cox method (Golgi-C.) and tested positive to the tyrosine hydroxylase by immunofluorescence (TH-immunofluorescence). Panel (a) shows a neuron simultaneously impregnated with the Golgi-Cox method and tested positive to TH-immunofluorescence. The results of the rendering are reported in panels (b) and (c), respectively separated for the TH-immunofluorescence and the Golgi-Cox impregnation. The co-localization analysis (d) clearly shows that the impregnation with Golgi-Cox is more detailed than the TH-immunofluorescence. In fact, the dendritic branching pointed to by the black arrow in panel (c) is not visualized in panel (d). From panel (e) it appears clear that the cell profile, size, and morphology can be appreciated better with the Golgi-Cox impregnation than with immunofluorescence; the white arrows indicate some spines-like dendritic structures that are only evident with mercuric impregnation. In panel (f) this stack is visualized rotated by 90° on the x-axis, to evaluate penetration of the primary and secondary anti-TH antibodies along the entire span of the scan.

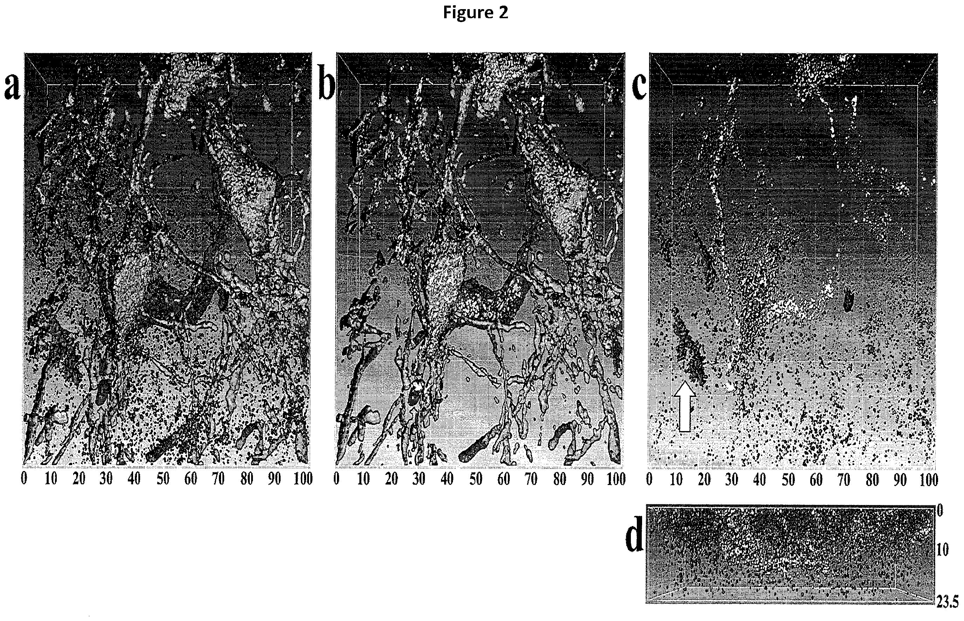

Figure 2.The figure shows mesencephalon neurons impregnated with the Golgi-Cox method, TH-positive and punctiform immunofluorescence for the Post-Synaptic-Density 95 proteins (PSD-95), surveyed with a surface rendering algorithm. In the figure, the distribution of the immunofluorescence of the PSD-95 co-localized with TH-positive neurons (b, c, d) is visible, with neurons impregnated with the Golgi-Cox method (b, c, d) and not co-localized (a, c, d). The white arrow points to the cellular profiles that are not stained but which are detectable through the distribution of the PSD-95. To evaluate the degree of penetration of the anti-PSD-96 antibodies (both primary and secondary), the stack has been rotated by 90° on axis z (d). The images in panels (c) and (d) have been obtained by unselected mapping of the channels for the TH-immunofluorescence and Golgi-Cox impregnation.

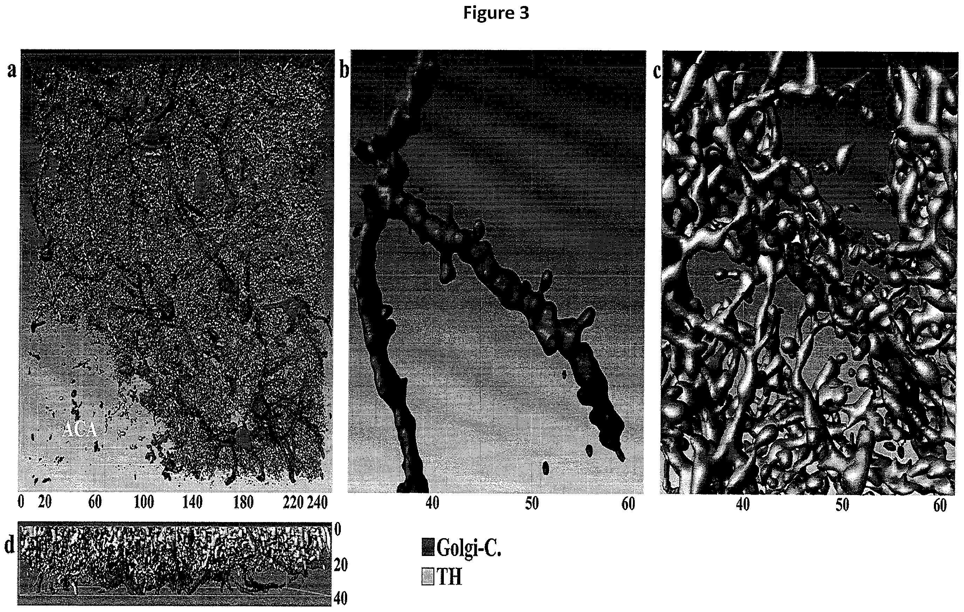

Figure 3. The figure shows the surface rendering in panel (a) of medium spiny neurons (MSN) of the striatum impregnated with the Golgi-Cox method adjacent to the anterior commissure (ACA) and surrounded by a very dense net of TH-positive fibers. This stack has been rotated by 90° to show the predominantly caudal-rostral orientation of the fibers (d). An MSN dendritic branch (b) is included in a dense net of TH-positive fibers which make close contacts with the dendritic trunk and the neck of the spines (c).

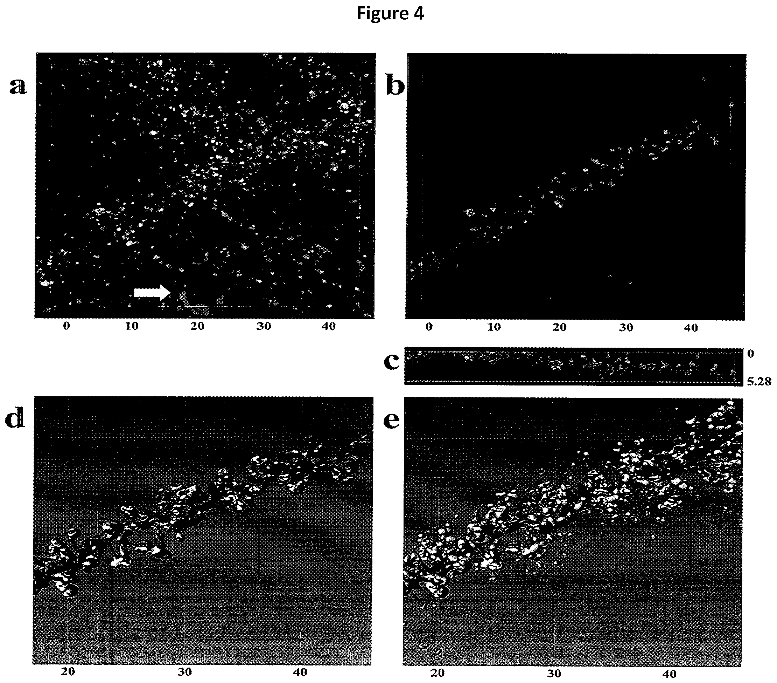

Figure 4. The figure shows the surface rendering of a dendritic branch of an MSN stained with the Golgi-Cox method and a double immunofluorescence for the anti-synapsin I (SynI) and the PSD-95. The co-localization analysis shows that the PSD-95 is especially distributed in the head of the spines, but also in their neck and in the dendritic trunk (b, c, d, e). When it was paired with the PSD-95, the immunofluorescence for the SynI indicated synapses, both symmetrical and asymmetrical, with the neck and the head of the spines respectively, and the dendritic trunk (a, e). Cellular profiles of non-stained neurons can also be seen in the striatum (white arrow). Panel (c) shows the same dataset rotated by 90° on the x axis without the SynI channel. In panel (e), some SynI points have been removed by hand to allow a better understanding of the relations between pre- and post-synaptic elements, with the dendrite stained with the Golgi-Cox method.

### Detailed description of the invention

As known and as already noted previously, Golgi's metal impregnation technique, including the variant of Golgi-Cox's mercuric impregnation, and the immunofluorescence technique cannot be applied in the same tissue section. This incompatibility essentially resides in the fact that for immunohistochemistry, the critical point is the step of tissue fixation, since denaturation of the sites for recognition of the antibodies and the diffusion of proteins from their initial localization must be prevented for avoiding the production of experimental artifacts and accordingly poor reliability of the experimental data.

To solve this technical problem, the inventors have developed a method which foresees fixation of the tissues *ex vivo* using aqueous solutions of paraformaldehyde in suitable concentrations, optionally pre-fixed with the same solution comprising paraformaldehyde. Of these two fixation steps the second, that is, the *ex vivo* fixation, is the critical one for the immunohistochemical staining, because for the staining of the tissues to be adequate, the antibodies have to penetrate in the entire depth of the tissues and to bind the specific antigens, in spite of the protein's fixation. The conditions for carrying out of this fixation are therefore essential to obtain an effective immunochemical staining. In this respect, although it is known that the protein fixing process with formaldehyde is slow and just about complete only in 24 hours (Helander KG, 1994, Biotech. Histochem. 69,177-179), it is not foregone that the latter will at the same time also allow an efficient penetration of the antibodies necessary for the immunohistochemistry and their actual binding with the antigens.

For the method of visualization and characterization of the nervous system by combining the staining for metal impregnation and immunocytochemistry, which is the object of the invention, the fixing step is therefore such to allow both fixation of the proteins and an efficient penetration of the antibodies for immunohistochemistry. More specifically, the fixation of nervous system specimens foresees a fixation of a tissue specimen to be examined with an aqueous solution comprising paraformaldehyde in a concentration between 1 and 2% w/v or a paraformaldehyde 4% w/v in Sorensen's Phosphate Buffer (PBS 0.4M, pH 7.4).

This method can, as a matter of fact, be carried out according to two different protocols, or rather, two different embodiments of the same method, which in any case foresee the fixation of specimens to be examined by immersion thereof at a temperature of 4°C for at least 8 hours to up to 12h in a paraformaldehyde solution 4% in PBS (0.4M, pH 7.4) followed by metal impregnation or in a composition comprising both the fixative, that is, the paraformaldehyde in concentration between 1 and 2% w/v, and the reagents for the metal impregnation, preferably according to the known Golgi-Cox method.

In the case, in which the fixation is carried out with an aqueous solution comprising paraformaldehyde between 1 and 2% w/v and the reagents for metal impregnation, the metal impregnation step is already started in the fixation step, and the subsequent impregnation step is carried out to renew the reagents and maximize impregnation. Essentially, in this second case also the fixative properties of the salt solution are exploited for the preferred Golgi-Cox mercuric impregnation, fixative properties which are moreover the basis for the metal impregnation itself, combined with the fixative properties of the paraformaldehyde. The composition that can be used for the combined fixation and metal impregnation step consists in an aqueous solution having pH 7.4 comprising:
- potassium dichromate (K₂Cr₂O₇) 5g
- mercuric chloride (HgCl₂) 5g
- potassium chromate (K₂CrO₄) 3.2g
- paraformaldehyde 5-10 g
- H₂O 480 mL.

Optionally, the step of fixation with paraformaldehyde 4% or with the composition comprising paraformaldehyde between 1 and 2% and the reagents for metal impregnation can be preceded by a pre-fixation by perfusion with the same solution comprising paraformaldehyde of the subsequent step of fixation *ex vivo.* This perfusion and pre-fixation step is not, however, essential to achieve the purpose of the invention and can therefore be omitted, thus allowing the application of the method also in the clinical field.

In relation to the metal impregnation technique and immunohistochemistry, in both protocols these are performed according to modes known to an expert in the field. Therefore, as known, the mercuric impregnation can be obtained by:
- immersion for 2 days in Golgi-Cox solution composed by: 5% potassium dichromate, 5% mercuric chloride, 5% potassium chromate (pH 6.5) (Glaser and van der Loos, 1981, J. Neuroscience Meth. 4, 117-125) in the dark;
- replacement of the Golgi-Cox solution with a fresh solution and maintenance of the specimens immersed in the dark for 14 days.

The immunohistochemistry, instead, is carried out on sections of impregnated tissue specimens and then, after metal, preferably mercuric, impregnation, the method comprises the further step of cutting the specimens in slices 50-100 µm thick with a cryostat or a vibratome. The sections obtained are then subjected to the following treatments before the immuno-staining in free floating with the suitable reagents:
- treatment of the slices with an ammonia aqueous solution 30% for 40 minutes in the dark at room temperature;
- fixing of the slices with a photographic fixative diluted 1:7 for 10 minutes.

In the case that the tissue is cut in slices with a cryostat, it must be treated before the slicing with a cryo-protective saccharose aqueous solution 30% by immersion for two or three days. This step can be skipped if a vibratome is used. Furthermore, when cutting with a cryostat, the temperature of the specimen is preferably of -4°C and the temperature of the blade -14°C. In any case, the slices are collected in pits that contain H₂O.

Furthermore, as known to an expert, the method according to the invention can comprise further steps, for example, a wash to remove the traces of fixatives used for the mercuric impregnation and for immunohistochemistry.

Typically, therefore, for what mentioned previously, according to an embodiment applicable in the experimental field, and when a greater quality of the visualization deriving from the immunohistochemistry is pursued, the method according to the invention comprises the steps of:
- fixing *ex vivo* the tissue specimens to be examined by immersion in a paraformaldehyde solution 4% in PBS (0.4M, pH 7.4) or an aqueous composition comprising paraformaldehyde 1-2% and the reagents for metal impregnation at 4°C for at least 8 hours, being said tissue previously pre-fixed by perfusion with the same fixation solution comprising paraformaldehyde;
- subjecting the fixed specimens to a metal impregnation, preferably a Golgi-Cox impregnation;
- treating slices of these specimens of impregnated tissues with the primary and secondary antibodies for the intended immunohistochemical reaction.

The embodiment of the method according to the invention without the pre-fixation of the tissue can be applied in clinical field.

In both cases, as known to an expert in the field, further steps must also be foreseen in between the main fixation steps, the metal impregnation and immunohistochemistry previously detailed. These steps are:
- washing of the brains in PBS for at least 8h (preferably 5 washes) to remove any trace of fixative after the *ex vivo* fixation;
- washing of the specimens with water after the metal impregnation;
- optionally, before the immuno-staining if the tissue is cut in slices with a cryostat, immersion for two or three days in a cryo-protective saccharose aqueous solution 30%;
- cutting of the tissue specimens in coronal slices 50-100 µm thick; with the cryostat the temperature of the specimen is preferably -4°C and the temperature of the blade -14°C; the slices are collected in pits that contain H₂O;
- after the treatment with ammonia, washing of the slices with distilled water for approximately 10 minutes;
- after the treatment with the photographic fixative, washing of the slices in distilled water (10 minutes), subsequently collected in PBS for immuno-staining;
- preliminary examination under the microscope of the metal impregnation; the neurons must appear black on a clear background;
- after the immuno-staining, examination of the doubly stained tissues with a conventional optical microscope and/or a confocal laser scanning microscope.

From the experimentation carried out, it has been possible to verify that the method of visualization and characterization of the nervous system, according to the invention, is in practice a simple method, easily reproducible which gives high-quality results and, therefore, fulfilling the established purposes.

The main advantages offered by this method can be summarized as follows:
- maximum visualization under optical microscope of the structural neuronal details;
- characterization of the antigens with specific antibodies and biochemical characterization of the impregnated neural elements of the same histological sections;
- visualization of the anatomical interactions between the neuronal elements highlighted in fluorescence and metal impregnation;
- possibility of using a confocal laser scanning microscope;
- saving on laboratory animals and sampling (without this method, the two procedures must be carried out separately on different animals/samples).

### EXPERIMENTAL PART

### Materials and methods

For the research described hereinafter, Sprague-Dawley male albino rats, supplied by Charles River (Como, Italy) with a weight of 200-225 g, have been used. All the experiments have been performed following the guidelines of the U.E. which regulates the use of experimental animals *(CEE N°86*/*609)* and the guidelines for the care and use of laboratory animals approved by the Italian Society for Neuroscience. Before any treatment, the animals were deeply anesthetized with urethane (1.3 g/kg) and subjected to transcardial perfusion with a physiological solution (saline solution 0.9%) or with Sorensen's Phosphate Buffer (PBS) 0.4M. The metallic impregnation has been carried out with a Golgi-Cox solution composed by: 5% potassium dichromate, 5% mercuric chloride, 5% potassium chromate (pH 6.5) (Glaser and van der Loos, 1981, *ref. cit*.).

The method of double staining by Golgi-Cox mercuric impregnation and immunohistochemistry has been validated on the "meso-cortico-limbic" and "nigrostriatal" nervous pathways that contain neurons and tyrosine hydroxylase (TH) positive fibers. To this end, the following reagents have been used: commercial primary poly and monoclonal antibodies of rabbit, and anti-tyrosine hydroxylase (TH) mice, anti post-synaptic-density 95 (PSD-95), and anti-synapsin I (SynI), respectively supplied by Santa Cruz Biotechnology Inc. (poly-clonal rabbit antibodies anti-TH (r_anti-TH) and anti-SynI; monoclonal mice antibodies anti-PDS95) and Sigma-Aldrich (monoclonal mice antibodies anti-TH (m_anti-TH)). The secondary antibodies used were: anti-mice IgM biotinylated goat and fluorescein-streptavidin (Vector Laboratories, Burlingame, CA), anti-rabbit Alexa Fluor 546 (1:200, Molecular Probes).

As a control procedure, three rats were deeply anesthetized and subjected to transcardial perfusion with a saline solution 0.9%, as above. The brains were immediately immersed in the Golgi-Cox solution, according to the traditional procedure, without any fixation and subsequent post-fixation with paraformaldehyde (Fregerslev S et al., 1971, Histochemie, 26, 289-304).

The fixation and staining protocol with mercuric impregnation and immunohistochemistry according to the method of the invention have been carried out according to the two ways of fixation *ex vivo* with paraformaldehyde 4% and mercuric impregnation in sequence or combined fixation and metal impregnation, described in detail hereinafter respectively in example 1 as method a) and in example 2 as method b).

### Example 1: fixation of cerebral tissues with paraformaldehyde 4% and mercuric impregnation carried out in sequence (method a).

Sprague-Dawley Charles River rats, (Como, Italy) with a weight of 200-225 were deeply anesthetized with chloral hydrate. Then, transcardial perfusion was carried out with 400µl of physiological solution, followed by 200µl of paraformaldehyde solution 4% in PBS (pH 7.4). The brains were carefully removed from the skull and placed in the same paraformaldehyde-based fixation solution at 4°C for the entire night. The brains were then washed in PBS for at least 8h (5 washes) to remove any trace of fixative.

Subsequently, each brain was immersed in the Golgi-Cox solution (in a quantity sufficient to entirely cover the specimens) and maintained in the dark for two days. The solution was then replaced with a fresh Golgi-Cox solution and the brain was maintained in it for a further 14 days, always in the dark.

The specimens were then washed with H₂O (3x5 min.) and transferred for two or three days in a cryo-protective saccharose solution 30%. The brains were then cut in coronal slices 50-100 µm thick. With the cryostat, the optimal temperature of the specimen is -4°C and the optimal temperature of the blade is -14°C. The slices obtained were collected in pits containing H₂O and treated with an ammonia solution 30% for 40 min. in the dark at room temperature. The slices were rinsed with distilled water for 10 min and fixed for 10 min. with a photographic fixative (Kodak Fix for Paper) diluted 1:7. Finally, the slices were rinsed in distilled water (10 min.) and collected in PBS for the subsequent immuno-staining in free floating. For the immunohistochemical staining, all the slices were washed in PBS (3 x 10 min.) at room temperature and, to prevent non-specific bonds, the slices were preincubated in a solution containing: 5% normal goat serum, 5% bovine serum albumin (BSA), and 0.5% Triton- X-100 in PBS at 4°C for the entire night. The slices were then incubated with the primary antibodies in the following combinations:
a) visualization of the tyrosine hydroxylase enzyme in slices impregnated in Golgi-Cox: m_anti-TH (1:400) in PBS for 48 h. at 4ºC;
b) visualization of the tyrosine hydroxylase enzyme and proteins of the PSD-95 in slices impregnated in Golgi-Cox: mixture of r_anti-TH (1:1000) and anti PSD-95 of mice (1:1000) in PBS for 48 h at 4ºC;
c) visualization of the tyrosine hydroxylase enzyme and proteins of the synapsin I in slices impregnated in Golgi-Cox: mixture of m_anti-TH (1:400) and anti-SynI of rabbit (1:1000) in PBS for 48 h at 4ºC;
d) visualization of the proteins of the PSD-95 and proteins of the synapsin I in slices impregnated in Golgi-Cox: mixture of anti-SynI of rabbit (1:1000) and anti PSD-95 of mice (1:1000) in PBS for 48 h at 4ºC.

All the sections were washed 3 x 10 min. in PBS and then incubated with the secondary antibodies according to the following pattern:
- the sections in a) anti-mice IgM biotinylated goat (1:200) in PBS for 4 h at room temperature; after this step, the slices were additionally washed in PBS (3 x 10 min) and treated with fluorescein-streptavidin (1:200) in PBS for 4h at room temperature;
- the sections in b), c), and d) anti-mice IgM biotinylated goat (1:200) in PBS for 4 h at room temperature; the slices were incubated with a mixture of fluorescein-streptavidin (1:200) and anti-rabbit Alexa Fluor 546 (1:200) in PBS for 4 h at room temperature.

At the end of the incubations with primary and secondary antibodies, all the sections were washed (3 x 10 min) in PBS, placed on a slide, and sealed with Vectashield (Vector Laboratories, Burlingame, CA).

### Example 2: fixation and metal impregnation carried out simultaneously (method b)

Method b) is the method which foresees combining of the fixation with paraformaldehyde and metal impregnation and, for this method, the composition already described previously is prepared. The preparation of this composition is described hereinafter.

### Preparation of the composition

The following aqueous solutions are prepared separately:
A) K₂Cr₂O₇ 5%;
B) HgCl₂ 5%;
C) K₂CrO₄ 4%.

Then, the different components are slowly mixed one with another and under continuous stirring as follows:
1. 200mL of water are added to 80 mL of solution C);
2. 10mL of solution A) are mixed with 10mL of solution B);
3. the mixtures obtained 1 and 2 are mixed and added with 5-10 g of paraformaldehyde. When the paraformaldehyde is added, the temperature must be monitored, because it must not exceed 60°C.

The pH of the solution thus obtained is controlled and, if necessary, it is brought to pH 7.4. The composition is then maintained in the dark for 5 days and then filtered.

### Fixation and impregnation protocol

The animals, the anesthesia, and the perfusion are as in example 1, with the difference that in this case, the pre-fixation by perfusion has been carried out with the composition comprising the paraformaldehyde and the reagents for metal impregnation prepared as described above. The perfusion with the composition has been followed by fixation of the brains *ex vivo* by immersion in the same composition. Continuation of the metal impregnation and immunofluorescence has been carried out as in example 1.

All the tissue specimens (examples 1 and 2) have been examined with conventional light microscopy to evaluate whether the impregnation was successful and with fluorescence microscopy and confocal laser scanning microscopy to evaluate whether the immunofluorescence reaction was successful. Both method a) of example 1 and method b) of example 2 according to the invention have been tested in the meso-cortico-limbic dopaminergic pathway, which contains several cerebral nuclei which go from the mesencephalon to the basal ganglia to the frontal cortex. In the mesencephalon (ventral tegmental area and substantia nigra) are the cellular bodies of the neurons containing dopamine (DA) which project their axons predominantly in the basal nuclei and in the frontal cortex. The expected results are, therefore, for the mesencephalon:
- visualization of the DA-ergic neurons in the mesencephalon, highlighted both by means of mercuric impregnation (Golgi-Cox) and of the immunofluorescence's reaction against the TH;
- visualization of the non-TH-positive neurons by means of Golgi-Cox staining;
- visualization of the pre- and post-synaptic proteins, both in the impregnated neurons and in those positive to immunofluorescence against the TH.

For the nigro-striatal area, instead, the expected results are:
- visualization of the TH-positive fibers, at least in part coming from the mesencephalon;
- visualization in Golgi-Cox of the neurons of the striatum (medium spiny neurons), which are the cells mainly targeted by DA-ergic neurons;
- visualization of the pre-synaptic proteins co-localized in the TH-positive terminals (immunofluorescence);
- visualization of the post-synaptic proteins co-localized in the medium spiny neurons (Golgi-Cox + immunofluorescence);
- interactions of these elements one with the other.

### Results

It was first of all surveyed that the pre-fixation and fixation *ex vivo* with paraformaldehyde 4%, or the pre-fixation by perfusion and fixation *ex vivo* with the composition comprising both paraformaldehyde and the reagents for the mercuric impregnation of the cerebral tissues do not prove to have significant effects on the standard Golgi-Cox mercuric impregnation, nor on the immunohistochemical staining.

In fact, in all the preparations examined with the Golgi-Cox procedure, a good impregnation of the neural elements was obtained, which proved homogenously distributed in the tissue. As a rule, only a portion of the total number of neurons proved stained, equal to approximately 5-10%. These neurons, distributed randomly and uniformly, resulted complete with dendritic tree, dendritic spines, and axons. Occasionally, also some glial elements and the lumen of some capillaries were stained. In all the impregnated slices examined under a conventional white light microscope, the stained elements appeared of an intense black color on clear background. The immunohistochemistry staining of the tissues stained with Golgi-Cox displayed very luminous fluorescent elements and, as expected, the penetration of all the antibodies was approximately 25-40 µm in both sides of the slice. However, immunoreactivity was only found in the sections coming from those brains which were perfused and post-fixed with paraformaldehyde, while no fluorescence was obtained in those brains that were only perfused with saline solution and fixed with Golgi-Cox solution alone (test control rats).

In particular, in the mesencephalon, the neurons impregnated with Golgi-Cox and those TH-positive (fluorescence) were simultaneously visualized with the confocal laser scanning microscope (CLSM). These neurons proved to be, as is customary, distributed over all the tissue, and did not seem to have been negatively affected by the previous impregnation. As a matter of fact, in all the preparations, the neurons always resulted complete with dendrites and axons, and the immunoreactivity surveyed was always homogenous.

Figure 1 shows both TH-positive neural elements and ones impregnated with Golgi-Cox in the Substantia Nigra compacta (SNc), and simultaneously visualized with the CLSM (1a). The figure shows that the application of these two procedures combined does not produce negative effects of one on the other but, on the contrary, supplies information on the neurochemical nature (TH-immuno-fluorescence alone, 1b) of the impregnated neurons (impregnation only, 1c). Furthermore, it was possible to verify that the Golgi-Cox impregnation offers a more detailed representation of the nervous structures than immunofluorescence alone: in fact, as shown in panels c and d of figure 1, the dendrite (black arrow) which appears in panel c cannot be appreciated with the TH-immuno-fluorescence (1b), as shown through the co-localization (1d). The depth of the scan, in this case, was 31.5 µm. This distance is consistent with the possibility of action of the antibodies (1f), which supports the interpretation of the result. The vector analysis shown in panel 1e, shows that the Golgi-Cox procedure provides a better representation of the morphological profile of the neuron on a whole, as for example the structures similar to dendritic spines (white arrows), as previously observed by Tepper et al. (Tepper JM et al., 1987, J. Neurosci. 7, 2794-2806). More specifically, the research carried out shows (figure 1e) how the morphological analyses based exclusively on the immunofluorescence for the TH can show minor precision.

The distribution of the immunoreactivity to the PSD-95 in the mesencephalon resulted not to be homogeneous. Figure 2 shows how the presence of PSD-95 can be visualized both in TH-positive neurons and in TH-negative ones, and simultaneously impregnated with the Golgi-Cox method. In accordance with Nowicka et al. (Nowicka D et al., 2003, Acta Neurobiol. Exp. 63, 185-195), the PSD-95 in panels 2a and 2c reveals a dense and punctiform staining which also allows one to identify the profiles of neuropils and dendrites of non-stained cells (white arrow).

In the striatum with Golgi-Cox staining and immunohistochemistry for the TH, numerous impregnated medium spiny neurons (MSN) were observed and immersed in a dense net of TH-positive fibers (figure 3a) coming from the mesencephalon. The surface rendering analysis has, as expected (Freund TF et al., 1984, Neuroscience 13, 1189-1215), shown that numerous TH-positive axon terminals form putative synaptic contacts predominantly with the neck of the spines and the dendritic trunk of the MSN (figure 3c).

Furthermore, the immunoreactivity with the PSD-95 and the SynI in the striatum was detected to be uniform. When the double immuno-staining was carried out with these antibodies in sections stained with the Golgi-Cox impregnation, it was possible to survey the relations between these two antigens in the dendrites and in the dendritic spines of the MSN (figure 4).In particular, clusters of PSD-95 were surveyed in association with the membrane of the MSN, while immunoreactivity of the SynI was always found outside the impregnated neurons (figure 4a).

In conclusion, the results obtained show that the method of visualization and characterization of the nervous system that is the object of the invention fulfills its purposes, since it has proven to be a simple and inexpensive method, suitable for both conventional microscopy and confocal microscopy, which grants the possibility to pair two techniques that are incompatible, that is, Golgi-Cox mercuric impregnation and immunohistochemistry. It has, instead, proven possible to combine these two techniques which have long been incompatible, for them to be used simultaneously in the same sections (Lee et al. 2006, *ref. cit*.), by following appropriate fixation procedures according to the method that is the object of the invention and, at the same time, giving optimal results extensively correspondent to the expected results based on the available knowledge in the field.

## Claims

1. A method for visualization and characterization of the nervous system **characterized by** combining the staining for metal impregnation and for immunocytochemistry comprising at least the steps of:
- fixing nervous tissue specimens with an aqueous solution comprising paraformaldehyde in a concentration comprised from 1 to 4% w/v;
- subjecting said fixed nervous tissue specimens to metal impregnation;
- treating cut sections of the metal impregnated tissue specimens with suitable reagents for the intended immune-histochemical staining.

2. The method for visualization and characterization of the nervous system according to claim 1, wherein the fixation is carried out with a 4% w/v paraformaldehyde solution in PBS (0.4 M, pH 7.4).

3. The method for visualization and characterization of the nervous system according to claim 1, wherein the fixation is carried out with a composition having pH 7.4 comprising:
- potassium dichromate 5 g
- mercuric chloride 5 g
- potassium chromate 3.2 g
- paraformaldehyde 5-10 g
- H₂O 480 mL.

4. The method for visualization and characterization of the nervous system according to one of the claims from 1 to 3, wherein the fixation is carried out on pre-fixed tissues with the same solution of fixation comprising paraformaldehyde in a concentration comprised from 1 to 4% w/v.

5. A composition comprising:
- potassium dichromate 5 g
- mercuric chloride 5 g
- potassium chromate 3.2 g
- paraformaldehyde 5-10 g
- H₂O 480 mL
having pH 7.4, for use in fixing tissue specimens according to the method defined in one of the claims from 1 to 4.

6. A kit for visualizing and characterizing the nervous system comprising at least one or more containers comprising a) a composition according to claim 5 and b) reagents for immunocytochemical staining.

7. The kit for the method according claim 6 further comprising one or more containers of a solution in PBS of paraformaldehyde 4% w/v.

## Patentansprüche

1. Verfahren zur Visualisierung und Charakterisierung des Nervensystems **gekennzeichnet durch** ein Verbinden des Färbens zur Metallimprägnierung und zur Immunozytochemie aufweisend zumindestens die Schritte:
- Fixieren von Nervengewebeproben mit einer wässrigen Lösung aufweisend Paraformaldehyd in einer Konzentration von 1 bis 4 Masse-%;
- Unterziehen der fixierten Nervengewebeproben einer Metallimprägnierung;
- Behandeln von Schnittbereichen der metallimprägnierten Gewebeproben mit einem geeigneten Reagenz zum geplanten immun-hystochemischen Färben.

2. Verfahren zur Visualisierung und Charakterisierung des Nervensystems gemäß Anspruch 1, wobei das Fixieren einer 4 Masse-prozentigen Paraformaldehydlösung in PBS (0,4 M, pH 7,4) ausgeführt wird.

3. Verfahren zur Visualisierung und Charakterisierung des Nervensystems nach Anspruch 1, wobei das Fixieren mit einer Zusammensetzung ausgeführt wird, welche einen pH 7,4 hat, aufweisend:
- Kaliumdichromat 5g
- Quecksilberchlorid 5g
- Kaliumchromat 3,2 g
- Paraformaldehyd 5 bis 10 g
- H₂O 480 ml.

4. Verfahren zur Visualisierung und Charakterisierung des Nervensystems nach einem der Ansprüche 1 bis 3, wobei das Fixieren mit vorfixierten Geweben mit der gleichen Fixierungslösung ausgeführt wird, aufweisend Paraformaldehyd in einer Konzentration aufweisend 1 bis 4 Masse-%.

5. Zusammensetzung aufweisend:
- Kaliumdichromat 5g
- Quecksilberchlorid 5g
- Kaliumchromat 3,2 g
- Paraformaldehyd 5 bis 10 g
- H₂O 480 ml
aufweisend einen pH 7,4 zur Verwendung zur Fixierung von Gewebeproben gemäß dem Verfahren, das in einem der Ansprüche 1 bis 4 definiert ist.

6. Ausrüstung zur Visualisierung und Charakterisierung des Nervensystems aufweisend zumindest einen oder mehrere Behälter aufweisend a) eine Zusammensetzung gemäß Anspruch 5 und b) Reagenzien für das immunozytochemische Färben.

7. Ausrüstung für das Verfahren gemäß Anspruch 6 weiter aufweisend einen oder mehrere Behälter mit einer Lösung in PBS von Paraformaldehyd 4 Masse-%.

## Revendications

1. Procédé pour la visualisation et la caractérisation du système nerveux **caractérisé par** la combinaison de la coloration pour l'imprégnation métallique et pour l'immunocytochimie comprenant au moins les étapes suivantes :
- la fixation des échantillons de tissu nerveux avec une solution aqueuse comprenant du paraformaldéhyde dans une concentration comprise entre 1 et 4 % p/v ;
- la soumission desdits échantillons de tissu nerveux fixés à une imprégnation métallique ;
- le traitement des coupes des échantillons tissulaires imprégnés de métal avec des réactifs appropriés pour la coloration immunohistochimique prévue.

2. Procédé pour la visualisation et la caractérisation du système nerveux selon la revendication 1, dans lequel la fixation est réalisée avec une solution de paraformaldéhyde à 4 % p/v dans du PBS (0,4 M, pH 7, 4) .

3. Procédé pour la visualisation et la caractérisation du système nerveux selon la revendication 1, dans lequel la fixation est réalisée avec une composition ayant un pH 7,4 comprenant :
- dichromate de potassium 5 g
- chlorure mercurique 5 g
- chromate de potassium 3,2 g
- paraformaldéhyde 5 à 10 g
- H₂O 480 ml.

4. Procédé pour la visualisation et la caractérisation du système nerveux selon l'une des revendications 1 à 3, dans lequel la fixation est réalisée sur des tissus préfixés avec la même solution de fixation comprenant du paraformaldéhyde dans une concentration comprise entre 1 et 4 % p/v.

5. Composition comprenant :
- dichromate de potassium 5 g
- chlorure mercurique 5 g
- chromate de potassium 3,2 g
- paraformaldéhyde 5 à 10 g
- H₂O 480 ml
ayant un pH 7,4, pour une utilisation dans la fixation d'échantillons tissulaires selon le procédé défini dans l'une des revendications 1 à 4.

6. Kit pour la visualisation et la caractérisation du système nerveux, comprenant au moins un ou plusieurs récipients comprenant a) une composition selon la revendication 5 et b) des réactifs pour la coloration immunocytochimique.

7. Kit pour le procédé selon la revendication 6 comprenant en outre un ou plusieurs récipients d'une solution dans du PBS de paraformaldéhyde à 4 % p/v.
